(19) Europäisches Patentamt
European Patent Office
Office européen des brevets

(11) Publication number: **0 034 385 B1**

# (12) EUROPEAN PATENT SPECIFICATION

(45) Date of publication of patent specification: **12.09.84**

(51) Int. Cl.³: **C 07 D 213/89,** A 61 K 7/06, A 61 K 7/08

(21) Application number: **81200125.3**

(22) Date of filing: **03.02.81**

## (54) Zinc-pyridinethione salts, a process for making thereof and hair care compositions containing them.

(30) Priority: **07.02.80 US 119347**
**07.02.80 US 119346**

(43) Date of publication of application:
**26.08.81 Bulletin 81/34**

(45) Publication of the grant of the patent:
**12.09.84 Bulletin 84/37**

(84) Designated Contracting States:
**AT BE CH DE FR GB IT LI LU NL SE**

(56) References cited:
**EP-A-0 007 704**
**US-A-2 809 971**
**US-A-3 723 325**
**US-A-3 773 770**

**INORGANIC CHEMISTRY, Vol. 16, No. 8, 1977 B.L. BARNETT et al. "Structural Characterization of Bis(n-oxopyridine-2-thionato)zinc(II)" pages 1834 to 1838**

(73) Proprietor: **THE PROCTER & GAMBLE COMPANY**
**301 East Sixth Street**
**Cincinnati Ohio 45202 (US)**

(72) Inventor: **Bolich, Raymond Edward, Jr.**
**7201 Striker Road**
**Maineville Ohio 45039 (US)**
Inventor: **Shaya, Steven Alan**
**9480 Maple Knoll Drive**
**Cincinnati Ohio 45239 (US)**
Inventor: **Steuri, Christian**
**4 St. James Court**
**Fairfield Ohio 45014 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 385 B1

## Description

The present invention relates to zinc salts of 1-hydroxy-2-pyridinethione which are in crystalline form and possess a geometry and particle size which make the crystals more compatible in a composition matrix, to a process for producing the zinc salts of 1-hydroxy-2-pyridinethione, and to hair care compositions containing them.

Pyridinethione salts are old as shown by US—A—2,809,971. Other patents disclosing similar compounds and processes for making them include US—A—2,786,847, US—A—3,583,999, US—A—3,590,035, and US—A—3,773,770.

The use of pyridinethione salts as antidandruff agents in shampoos and hair rinses is well known. US—A—3,236,733 discloses detergent compositions containing such salts. Barnett, B L et al, "Structural Characterization of Bis (N-oxypyridine-2-thionato)Zinc(II)," *Inorganic Chemistry, 16,* 1834, (1977) discloses recrystallizing zinc pyridinethione crystals from chloroform or dimethyl sulfoxide. Other references which disclose pyridinethione salts are US—A—2,809,971, US—A—3,723,325, US—A—3,753,916, US—A—3,761,417, US—A—3,761,418.

While it is known to use pyridinethione salts in hair care compositions the prior art does not indicate whether the salts are compatible with all components which may be present in a composition. Included among components which may be present in shampoos and other hair care compositions are those which make the compositions more aesthetically appealing. Aesthetics are important in encouraging a consumer to purchase a product as well as continue using the product after purchase.

One aesthetic characteristic which has found wide acceptance is pearlescence. This is quite often achieved through the use of material such as ethylene glycol distearate. The pyridinethione salt materials available up to this time have substantially interfered with the ability of pearlescent material to deliver acceptable pearlescence. This has been due, in all likelihood, to the materials being in the shape of cubes and rods and of small size. The recrystallized material of Barnett et al supra, while allowing pearlescence, has been found to be less acceptable than the crystals of the present invention in finished compositions.

Furthermore, while the prior art discloses pyridinethione salts and processes for making such salts, it does not suggest forming the salts in an aqueous surfactant medium. Furthermore, there is no suggestion that the crystals formed in such a medium would have superior aesthetic properties and be more compatible in a composition.

It is therefore an object of the present invention to provide zinc pyridinethione salt crystals which interfere less with the pearlescent material than do prior art materials.

It is another object of the present invention to provide antidandruff compositions containing such crystals.

It is yet another object of the present invention to provide an improved method for making zinc pyridinethione salt crystals.

These and other objects will become obvious from the detailed description which follows.

### Disclosure of the Invention

The present invention relates to zinc pyridinethione salt crystals which are predominantly flat platelets having a mean sphericity in the range from 0.2 to 0.65 and a median particle size of at least 2 $\mu$m but less than 15 $\mu$m diameter, expressed as the equivalent diameter of a sphere of equal volume. Compositions containing such crystals are also part of the present invention.

The present invention further relates to the formation of zinc pyridinethione salt crystals by reacting pyridinethione or a water soluble zinc salt in an aqueous surfactant medium. The concentration of surfactant is not critical but is preferably from 1% to 24%, more preferably from 1% to 8%, by weight. The reaction temperature is at least 20°C., preferably from 60°C. to 100°C. The crystals may be separated from the reaction medium after formation for use in a particular formation not containing the surfactant used in the reaction. Alternatively, if desired, the crystals may be left in the reaction medium for incorporation into a final composition.

### Detailed Description of the Invention

#### Pyridinethione Salt Crystals

The present invention is concerned with zinc salts of 1-hydroxy-2-pyridinethione which has the following structural formula in tautomeric form, the sulfur being attached to the No. 2 position in the pyridine ring.

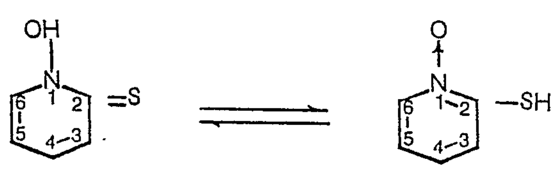

The zinc salts represent substitution of the metal cation for the hydrogen of one of the tautomeric forms.

The present pyridinethione salt crystals are predominantly flat platelets which have a mean sphericity between 0.20 and 0.65 and a median particle size of at least 2 $\mu$m but less than 15 $\mu$m diameter, expressed as the median equivalent diameter of a sphere of equal volume. The median diameters are on a mass basis with 50% of the mass of particles falling on either side of the value given.

The diameter of a sphere of equivalent volume for a particle can be determined by a variety of sedimentation techniques which are based on Stokes' Law for the settling velocity of a particle in a fluid. Such techniques are described in Stockham, J. D. and Fochtman, E. G., *Particle Size Analysis,* Ann Arbor Science, 1978. An approach for determining the median equivalent spherical diameter based on volume, $\bar{d}_v$, is shown in Example II.

The sphericity of a particle is also described by Stockham and Fochtman at page 113 as

$$\psi = \left(\frac{d_v}{d_s}\right)^2$$

where $d_v$ is the diameter of a sphere of equivalent volume, supra, and $d_s$ is the diameter of a sphere of equivalent area. In the present invention the mean sphericity =

$$\left(\frac{\bar{d}_v}{\bar{d}_s}\right)^2$$

or surface area of spheres having equivalent volume distribution divided by the actual surface area of particles as measured. A technique for determining actual surface area is shown in the examples using the BET technique described by Stockham and Fochtman at page 122.

Compositions Containing the Pyridinethione Metal Salts

The pyridinethione metal salts of the present invention are useful in a variety of hair care compositions as antidandruff aids. The salts are generally present at a level of from 0.2% to 4.0% by weight, preferably from 0.3% to 2.0%, even more preferably from 1% to 2% in some compositions. Included among the hair care compositions are shampoos, creme rinses, hair tonics and many others. Shampoos are the preferred compositions and components generally found in such compositions are given below.

Surfactant

One essential component of the antidandruff shampoos herein is a surfactant. The term "surfactant" as used herein is intended to denote soap and nonsoap surfactants. The surfactant component comprises from 10% to 50% by weight of the composition, preferably from 10% to 20%.

Any nonsoap surfactant is suitable for use including anionic, nonionic, amphoteric and zwitterionic types. Cationic surfactants may also be used, but these are not preferred in the shampoos of the present invention due to their irritation potential.

Examples of suitable soaps are the sodium, potassium, ammonium and alkanol ammonium salts of higher fatty acids (those having 10—20 carbon atoms). Anionic nonsoap surfactants can be exemplified by the alkali metal salts of organic sulfuric reaction products having in their molecular structure an alkyl radical containing from 8—22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$—$C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and other known in the art.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compound containing from 40% to 80% polyoxyethylene by weight and having a molecular weight of from 5,000 to 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconut alcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$$R_1R_2R_3N \rightarrow O$$

wherein

$R_1$ contains an alkyl, alkenyl or monohydroxy alkyl radical of from 8 to 18 carbon atoms, from 0 to 10 ethylene oxide moieties, and from 0 to 1 glyceryl moiety, and $R_2$ and $R_3$ contain from 1 to 3 carbon atoms and from 0 to 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl)amine oxide, dimethyloctylamine oxide, dimethyldecylamine oxide, dimethyltetradecylamine oxide, 3,6,9-trioxaheptadecyldiethylamine oxide, di-(2-hydroxyethyl)-tetradecylamine oxide, 2-dodecoxyethyldimethylamine oxide, 3-dodexocy-2-hydroxypropyldi(3-hydroxypropyl)amine oxide, dimethylhexadecylamine oxide.

5. Long chain tertiary phosphine oxides corresponding to the following general formula:

$$RR'R''P \rightarrow O$$

wherein

R contains an alkyl, alkenyl or monohydroxyalkyl radical ranging from 8 to 18 carbon atoms in chain length, from 0 to 10 ethylene oxide moieties and from 0 to 1 glyceryl moiety and R′ and R″ are each alkyl or monohydroxyalkyl groups containing from 1 to 3 carbon atoms. The arrow in the formula is a conventional representation of a semipolar bond. Examples of suitable phosphine oxides are:
dodecyldimethylphosphine oxide,
tetradecyldimethylphosphine oxide,
tetradecylmethylethylphosphine oxide,
3,6,9-trioxaoctadecyldimethylphosphine oxide,
cetyldimethylphosphine oxide,
3-dodecoxy-2-hydroxypropyldi(2-hydroxyethyl)phosphine oxide,
stearyldimethylphosphine oxide,
cetylethylpropylphosphine oxide,
oleyldiethylphosphine oxide,
dodecyldiethylphosphine oxide,
tetradecyldiethylphosphine oxide,
dodecyldipropylphosphine oxide,
dodecyldi(hydroxymethyl)phosphine oxide,
dodecyldi(2-hydroxyethyl)phosphine oxide,
tetradecylmethyl-2-hydroxypropylphosphine oxide,
oleyldimethylphosphine oxide,
2-hydroxydodecyldimethylphosphine oxide.

6. Long chain dialkyl sulfoxides containing one short chain alkyl or hydroxy alkyl radical of 1 to 3 carbon atoms (usually methyl) and one long hydrophobic chain which contain alkyl, alkenyl, hydroxy alkyl, or keto alkyl radicals containing from 8 to 20 carbon atoms, from 0 to 10 ethylene oxide moieties and from 0 to 1 glyceryl moiety. Examples include:
octadecyl methyl sulfoxide,
2-ketotridecyl methyl sulfoxide,
3,6,9-trioxaoctadecyl 2-hydroxyethyl sulfoxide,
dodecyl methyl sulfoxide,
oleyl 3-hydroxypropyl sulfoxide,

tetradecyl methyl sulfoxide,
3-methoxytridecyl methyl sulfoxide,
3-hydroxytridecyl methyl sulfoxide,
3-hydroxy-4-dodecoxybutyl methyl sulfoxide.

Zwitterionic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from 8 to 18 carbon atoms and one contains an anionic water-solubilizing group, e.g., carboxy, sulfonate, sulfate, phosphate, or phosphonate. A general formula for these compounds is:

$$R^2-\overset{\displaystyle (R^3)_x \atop |}{Y^{(+)}}-CH_2-R^4-Z^{(-)}$$

wherein

$R^2$ contains an alkyl, alkenyl, or hydroxy alkyl radical of from 8 to 18 carbon atoms, from 0 to 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to 3 carbon atoms; X is 1 when Y is a sulfur atom and 2 when Y is a nitrogen or phosphorus atom; $R^4$ is an alkylene or hydroxyalkylene of from 1 to 4 carbon atoms and Z is a radical selected from the group consisting of carboxylate, sulfonate, sulfate, phosphonate, and phosphate groups.

Examples include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]butane-1-carboxylate;
3-[S-ethyl-S-(3-dodecoxy-2-hydroxypropyl)sulfonio]propane-1-phosphate;
3-(P,P-dimethyl-P-dodecylphosphonio)-propane-1-phosphonate; and
5-[N,N-di(3-hydroxypropyl)-N-hexadecylammonio]-2-hydroxypentane-1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from 8 to 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines such as the one prepared by reacting dodecylamine with sodium isethionate according to the teaching of US—A—2,658,072, N-higher alkyl aspartic acids such as those produced according to the teaching US—A—2,438,091, and the products sold under the trade name "Miranol" and described in US—A—2,528,378.

Many cationic surfactants are known to the art. By way of example, the following may be mentioned:
dodecyltrimethylammonium chloride;
nonylbenzylethyldimethylammonium nitrate;
tetradecylpyridinium bromide;
laurylpyridinium chloride;
cetylpyridinium chloride;
laurylpyridinium chloride;
laurylisoquinolium bromide;
ditallow (hydrogenated) dimethyl ammonium chloride
dilauryldimethylammonium chloride; and
stearalkonium chloride.

Many additional nonsoap surfactants are described in McCUTCHEON'S, DETERGENTS AND EMULSIFIERS, 1979 ANNUAL, published by Allured Publishing Corporation.

The above-mentioned surfactants can be used alone or in combination in the shampoo compositions of the present invention.

## Pearlescent Material

Another desirable component of the shampoos herein is a pearlescent material. Such materials are well known in the art and include bismuth oxychloride, stearic monoethanolamide, ethylene glycol monostearate or distearate, guanine and titanium dioxide coated mica. A pearlescent material is generally present at a level of from 10.1% to 6%, preferably from 0.5% to 5%, by weight.

Aqueous Carrier

The shampoos herein are preferably in the form of liquids or creams in which water is the principal diluent. The level of water in the compositions is typically from 35% to 90% by weight.

Optional Components

The antidandruff shampoos herein can contain a variety of non-essential optional ingredients suitable for rendering such compositions more stable and desirable. Such conventional optional ingredients are well known to those skilled in the art, e.g., preservatives such as benzyl alcohol, methyl paraben, propyl paraben and imidazolidinyl urea; thickeners and viscosity modifiers such as coconut ethanol amide, sodium chloride, sodium sulfate, carboxymethylcellulose, methylcellulose, polyvinyl alcohol, and ethyl alcohol; pH adjusting agents such as citric acid, succinic acid, phosphoric acid, sodium hydroxide, sodium carbonate, etc.; suspending agents such as magnesium/aluminum silicate; perfumes, dyes; and sequestering agents such as disodium ethylenediamine tetraacetate.

Minor ingredients such as perfumes, dyes and coloring agents can also be added to the instant compositions to improve their consumer acceptability. If present, such agents generally comprise from 0.1% to 2.0% by weight of the composition. The pH of the shampoos herein is generally from 3 to 9.

Other Hair Care Compositions

Creme rinses, hair tonics and other hair care compositions as well as shampoos may contain the pyridinethione salts of the present invention. These compositions may contain a variety of other components including some of those described supra for shampoos. Creme rinses generally contain a cationic surfactant similar to those described supra, particularly stearalkonium chloride and ditallow dimethyl ammonium chloride, at a level of from 0.1% to 5%, preferably from 0.5% to 2%, by weight.

METHOD OF MANUFACTURE

The hair care compositions containing the pyridinethione salt crystals may be made using techniques well known in the art.

The zinc pyridinethione salt crystals used in the compositions of this invention are prepared by a process which involves the steps enumerated herein above. The reactants can be handled in a wide variety of ways. For example, the water soluble zinc salt may be combined with part of the surfactant and water while the remainder of the water and surfactant are combined with pyridinethione or a water soluble pyridinethione salt. The two mixtures are then combined. Alternatively, all of the surfactant may be combined with either material or put into a third container into which the materials are then placed. All of these approaches are satisfactory since the crystals form almost instantaneously regardless of the approach used.

The various reaction materials are discussed in detail below.

Pyridinethione and Water Soluble Pyridinethione Salts

Pyridinethione as the term is used herein is 1-hydroxy-2-pyridinethione which has the following structural formula in tautomeric form, the sulfuric being attached to the No. 2 position in the pyridine ring.

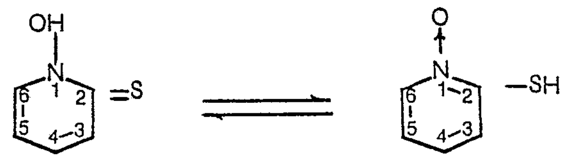

The water soluble salts represent substitution of the metal cation for the hydrogen of one of the tautomeric forms. The preferred salts are those involving ammonium or alkali metals. Most preferred is the sodium salt.

The amount of 1-hydroxy-2-pyridinethione or water soluble salt can vary over a wide range (the level only dependent on the quantity of material desired). A preferred level is from 8% to 16% by weight of the total reaction system.

Water Soluble Zinc Salt

Suitable zinc salts may be nitrates, acetates, sulfates or halides. The preferred salts are sulfates. The amounts of soluble zinc salt used is not critical so long as the amount is sufficient to form the desired pyridinethione salt. The amount used therefore is generally an amount sufficient to provide a stoichiometric excess. An excess of 5% by weight is an example of a suitable excess amount.

Surfactant

Another necessary component in the process of the present invention is a surfactant. The term "surfactant" as used herein is intended to denote soap and non-soap surfactants. Any nonsoap

surfactant is suitable for use including anionic, nonionic, amphoteric, cationic and zwitterionic types. The surfactant should be soluble in water at the temperature of the reaction.

Examples of surfactants suitable for use in the process of this invention are the same surfactants which are described herein above as examples of surfactants suitable for use in the compositions of the invention herein. These surfactants can be used alone or in combination in the process herein. As noted above in the description of the process of this invention, surfactant is preferably used in the process herein at a concentration of from 1% to 24%, more preferably from 1% to 8%, by weight and surfactant used in the process herein should be soluble in water at the temperature of the reaction.

Reaction Temperature

The reaction temperature should be at least 20°C., preferably from 60°C. to 100°C. The temperature can be achieved and maintained through the use of any of the techniques well known in the art.

Reactant Medium

The process of the present invention is carried out in an aqueous medium wherein water is the major diluent.

## INDUSTRIAL APPLICABILITY

The pyridinethione salts and compositions containing them are useful in combatting dandruff. Such compositions are used in a conventional manner.

The following Examples further describe and demonstrate the preferred embodiments within the scope of the present invention. Unless otherwise indicated, all percentages herein are by weight.

### Example I

Zinc pyridinethione salt crystals of the present invention were made using the following procedure.

A first mixture was prepared by combining 14.7 parts of zinc sulfate, 21.7 parts of a 29% aqueous solution of sodium alkyl sulfate and 8.6 parts of water in a mix tank. This mixture was heated to 95°C.

A second mixture was prepared by combining 35 parts of a 40% aqueous solution of sodium pyridinethione with 20 parts of a 29% aqueous solution of sodium alkyl sulfate in a mix tank. This second mixture was also heated to 95°C.

The first mixture was added to the second mixture resulting in the formation of zinc pyridinethione crystals which were washed and collected.

The total batch size, the combined mixtures, was 2500 grams.

### Example II

The crystals of zinc pyridinethione made according to Example I were evaluated to determine their particle size and sphericity.

The median equivalent spherical diameter based on volume (particle size) was determined by means of a SediGraph 500 D Particle Size Analyzer supplied by Micrometrics Instrument Corporation.

The SediGraph 5000 D determines, by means of X-ray absorption, the concentration of particles remaining at decreasing sedimentation depths as a function of time. Stokes' Law relates the measured equilibrium velocity of a particle falling through a viscous medium to its equivalent spherical diameter (ESD).

$$ESD = \left[\frac{18 \cdot \eta \cdot \nu}{(\rho - \rho_0)g}\right]^{1/2}$$

$\eta$ = liquid viscosity
$\nu$ = equilibrium velocity
$\rho_o$ = liquid density
g = gravitational acceleration
$\rho$ = particle density

$\nu$ is determined from the Sedigraph while the other variables are available from reference sources or obtained experimentally. In the present analysis water was the liquid and the liquid viscosity was $0.76 \times 10^{-3}$ Pa.s (0.76 cp).

The density of ZPT particles is known to be about 1.81 g/cc, (Barnett, B. L., et al, "Structural Characterization of Bis-(N-oxypyridine-2-thionato) Zinc (II)", *Inorganic Chemistry 16,* 1934, [1977]).

The median equivalent spherical diameter based on volume ($\bar{d}_v$) of the crystals using Stokes' Law was determined to be 5.4 $\mu$.

This median equivalent spherical diameter was taken from the mass distribution of particles

described in 1. below. The determination of a specific surface area based on equivalent spherical diameters was as follows:

1. A cumulative mass distribution of equivalent spherical diameters in μm was obtained using the SediGraph instrument described previously. The rate for the instrument was 866 and the starting diameter of 100 μm.

2. The cumulative mass distribution was divided into equal logarithmic intervals in μm. The sizes of the intervals are shown in the following table.

3. The cumulative mass distribution at each equal logarithmic interval was determined.

4. The diameter at the centerpoint of each interval was determined.

5. The value of the cumulative mass percent distribution at the centerpoints of the intervals was determined.

6. The value of the differential mass percent distribution was then determined.

7. The amount of material for each interval was determined, assuming that there was a total of one gram which was evaluated. These values are the values in 6, above, divided by 100.

8. The assumed spherical particle surface area of the material contained in each interval was calculated using diameters equal to the centerpoints of the intervals. Therefore, the calculation for a particular interval, i, is

$$A_i = \frac{\text{(Mass contained in interval i)}}{\text{(Mass of sphere with diameter } d_i)} \times$$

$$\text{(Surface area of sphere with diameter } d_i)$$

$$A_i = \frac{\text{Mass contained in interval i}}{\rho\ 4/3\ \pi\left(\frac{d_i}{2}\right)^3}\left(d_i\right)^2\pi = \frac{6}{\rho}\ \frac{\text{mass}}{d_i}$$

$$A = \Sigma_i A_i = \text{Surface area/g assuming spheres}$$

All of the above data are shown in the following table. The numerical column headings correspond to the numbers above.

| 2 | 3 | 4 | 5 | 6 | 7 | 8 |
|---|---|---|---|---|---|---|
| 1.59 | 0. | | | | | |
| | | 1.78 | 0.5 | 0.5 | 0.005 | 0.009 |
| 2.00 | 1.0 | | | | | |
| | | 2.28 | 2.5 | 2.0 | 0.020 | 0.029 |
| 2.52 | 4.0 | | | | | |
| | | 2.87 | 7.0 | 4.5 | 0.045 | 0.052 |
| 3.17 | 10.0 | | | | | |
| | | 3.59 | 16.0 | 9.0 | 0.090 | 0.083 |
| 4.00 | 22.0 | | | | | |
| | | 4.52 | 32.0 | 16.0 | 0.160 | 0.118 |
| 5.04 | 42.0 | | | | | |
| | | 5.70 | 55.0 | 23.0 | 0.230 | 0.134 |
| 6.35 | 68.0 | | | | | |
| | | 7.18 | 76.0 | 21.0 | 0.210 | 0.097 |
| 8.00 | 84.0 | | | | | |
| | | 9.04 | 88.5 | 12.5 | 0.125 | 0.046 |
| 10.08 | 93.0 | | | | | |
| | | 11.4 | 95.0 | 6.5 | 0.065 | 0.019 |
| 12.7 | 97.0 | | | | | |
| | | 14.4 | 98.0 | 3.0 | 0.030 | 0.007 |
| 16.0 | 99.0 | | | | | |
| | | 18.1 | 99.5 | 1.5 | 0.015 | 0.003 |
| 20.2 | 100.0 | | | | | |
| | | 22.8 | 100.0 | 0.5 | 0.005 | 0.001 |
| 25.4 | 100.0 | | | | | |
| | | | TOTAL – 100.0 | | 1.000g | 0.598 $m^2/g$ |

The specific surface area for the crystals was determined by means of a B.E.T. surface area analysis using nitrogen gas. The B.E.T. analysis showed the crystals to have a specific surface area of 2.39 $m^2/g$.

The sphericity was then determined as follows:

$$\psi = \frac{\text{area per g calculated for assumed spherical distribution}}{\text{area per g from B.E.T. measurement}}$$

$$\psi = \frac{0.598}{2.39} = 0.25$$

Example III

A shampoo composition having the following formula was prepared.

| Component | Level |
|---|---|
| Ammonium Alkyl Sulfate (29% Aqueous solution) | 55.25% |
| Zinc Pyridinethione Crystals of Example I | 2.0 |
| Coconut Monoethanolamide | 3.0 |
| Ethylene Glycol Distearate | 5.0 |
| Sodium Citrate | 0.5 |
| Citric Acid | 0.2 |
| Color Solution | 0.1 |
| Perfume | 0.5 |
| Water | q.s. 100.00% |

The pearlescence of this composition was better than an identical composition containing prior art zinc pyridinethione particles having a mean sphericity of about 0.75 and a mean equivalent spherical diameter based on volume of about 1.25 $\mu$.

Example IV

The following shampoo is prepared.

| Component | Level |
|---|---|
| Triethanolamine Alkyl Sulfate (35% Aqueous Solution) | 50.0% |
| Triethanolamine | 3.0 |
| Coconut Monoethanolamide | 2.0 |
| Magnesium/Aluminum Silicate | 0.5 |
| Hydroxy Methyl Cellulose | 0.6 |
| Zinc Pyridinethione crystals of Example I | 2.0 |
| Color Solution | 0.1 |
| Perfume | 0.3 |
| Water | q.s. 100.0% |

Example V

The following cream shampoo is prepared.

| Component | Level |
|---|---|
| Sodium Alkyl Glyceryl Sulfonate | 60.0% |
| Sodium Chloride | 5.0 |
| Sodium N-Lauroyl Sarcosinate | 12.0 |
| N-Cocoyl Sarcosine Acid | 1.0 |
| Lauric Dethanolamide | 2.0 |
| Zinc Pyridinethione of Example I | 2.0 |
| Perfume | 0.5 |
| Color Solution | 0.12 |
| Water | q.s. 100.00% |

Example VI

The following creme rinse is prepared.

| Component | Level |
|---|---|
| Stearalkonium Chloride | 0.75% |
| Stearyl Alcohol | 0.5 |
| Zinc Pyridinethione of Example I | 0.3 |
| Cetyl Alcohol | 0.5 |
| Polyoxyethylene (2) Cetyl Ether | 0.8 |
| Sodium Chloride | 0.25 |
| Color Solution | 0.2 |
| Perfume | 0.25 |
| Water | q.s. 100.00% |

Example VII

Zinc pyridinethione salt crystals were made using the following process of the present invention.

A first mixture was prepared by combining 7.3 parts of a 28% aqueous solution of ammonium alkyl sulfate, 2 parts of zinc sulfate and 40.7 parts of water in one mix tank. The mixture was heated to 80°C.

A second mixture was prepared by combining 7.3 parts of a 28% aqueous solution of ammonium alkyl sulfate, 5 parts of a 40% aqueous solution of sodium pyridinethione and 37.7 parts of water in another mix tank. This mixture was also heated to 80°C.

The first and second mixtures were metered into a third tank at the rate of 1 kg./min. The resulting zinc pyridinethione crystals were washed and stored.

**Claims**

1. A zinc salt of 1-hydroxy-2-pyridinethione characterised by being in the form of predominantly flat platelet crystals having a mean sphericity in the range from 0.2 to 0.65 and a median equivalent spherical diameter based on volume of at least 2 $\mu$m but less than 15 $\mu$m.

2. A hair care composition characterised in that it contains from 0.2% to 4.0% by weight of a pyridinethione salt according to Claim 1.

3. A hair care composition according to Claim 2 characterised by being in the form of a shampoo

which additionally contains from 10% to 50% by weight of a surfactant.

4. A shampoo according to Claim 3 characterised in that it additionally contains from 0.1% to 6.0% by weight of a pearlescent material.

5. A hair care composition according to Claim 2 characterised by being in the form of a creme rinse which additionally contains from 0.1% to 5.0% by weight of a cationic surfactant.

6. A process for making a zinc pyridinethione salt according to Claim 1 characterised by reacting 1-hydroxy-2-pyridinethione or a water soluble salt thereof with a water soluble zinc salt in an aqueous surfactant medium, wherein the reaction temperature is at least 20°C.

7. A process according to Claim 6 characterised in that the pyridinethione compound is an alkali metal pyridinethione salt and the concentration of surfactant in the total reaction mixture is from 1% to 24% by weight.

8. A process according to Claim 6 or 7 characterised in that the zinc salt is present in an amount sufficient to provide a stoichiometric excess.

9. A process according to any of Claims 6 to 8 characterised by reacting sodium 1-hydroxy-2-pyridinethione with zinc sulfate in an aqueous anionic surfactant medium at a temperature in the range from 60°C to 100°C, wherein the concentration of surfactant in the total reaction mixture is from 1% to 8% by weight.

**Patentansprüche**

1. Ein Zinksalz von 1-Hydroxy-2-pyridinthion, dadurch gekennzeichnet, daß es in der Form von überwiegend flachen, plättchenförmigen Kristallen mit einer Durchschnittskugelgestalt im Bereich von 0,2 bis 0,65 und einem äquivalenten Kugeldurchmesser der Mittellinie, auf Basis des Volumens, von wenigtens 2 $\mu$m, aber weniger als 15 $\mu$m, vorliegt.

2. Eine Haarpflegezusammensetzung, dadurch gekennzeichnet, daß sie 0,2 Gew.-% bis 4,0 Gew.-% eines Pyridinthionsalzes nach Anspruch 1 enthält.

3. Eine Haarpflegezusammensetzung nach Anspruch 2, dadurch gekennzeichnet, daß sie in der Form eines Shampoos vorliegt, welches zusätzlich 10 Gew.-% bis 50 Gew.-% eines oberflächenaktiven Mittels enthält.

4. Ein Shampoo nach Anspruch 3, dadurch gekennzeichnet, daß es zusätzlich 0,1 Gew.-% 6,0 Gew.-% eines perlglänzenden Materials enthält.

5. Eine Haarpflegezusammensetzung nach Anspruch 2, dadurch gekennzeichnet, daß sie in der Form einer Cremespülung vorliegt, welche zusätzlich 0,1 Gew.-% bis 5,0 Gew.-% eines kationischen, oberflächenaktiven Mittels enthält.

6. Ein Verfahren zur Herstellung eines Zinkpyridinthionsalzes nach Anspruch 1, dadurch gekennzeichnet, daß 1-Hydroxy-2-pyridinthion, oder ein wasserlösliches Salz davon mit einem wasserlöslichen Zinksalz in einem wässerigen, oberflächenaktives Mittel enthaltenden Medium, worin die Reaktionstemperatur wenigstens 20°C beträgt, umgesetzt wird.

7. Ein Verfahren nach Anspruch 6, dadurch gekennzeichnet, daß die Pyridinthion verbindung ein Alkalimetallpyridinthionsalz ist und die Konzentration an oberflächenaktivem Mittel in dem gesamten Reaktionsgemisch 1 Gew.-% bis 24 Gew.-% beträgt.

8. Ein Verfahren nach Anspruch 6 oder 7, dadurch gekennzeichnet, daß das Zinksalz in einer zur Schaffung eines stöchiometrischen Überschusses ausreichenden Menge vorliegt.

9. Ein Verfahren nach irgendeinem der Ansprüche 6 bis 8, dadurch gekennzeichnet, daß Natrium-1-hydroxy-2-pyridinthion mit Zinksulfat in einem wässerigen, anionisches, oberflächenaktives Mittel enthaltenden Medium, bei einer Temperatur im Bereich von 60°C bis 100°C, bei einer Konzentration von oberflächenaktivem Mittel in dem gesamten Reaktionsgemisch von 1 Gew.-% bis 8 Gew.-%, umgesetzt wird.

**Revendications**

1. Sel de zinc de la 1-hydroxy-2-pyridinethione caractérisé en ce qu'il se trouve pour la plus grande partie sous forme de cristaux en plaquettes aplaties ayant une sphéricité moyenne de 0,2 à 0,65 et un diamètre médian de sphère équivalente, calculé d'après le volume, d'au moins 2 $\mu$m, mais inférieur à 15 $\mu$m.

2. Composition capillaire caractérisée en ce qu'elle contient 0,2 à 4,0% en poids d'un sel de pyridinethione selon la revendication 1.

3. Composition capillaire selon la revendication 2, caractérisée en ce qu'elle se présente sous forme d'un shampooing qui contient en outre 10 à 50% en poids de tensio-actif.

4. Shampooing selon la revendication 3, caractérisé en ce qu'il contient en outre 0,1 à 6,0% en poids de substance nacrée.

5. Composition capillaire selon la revendication 2, caractérisée en ce qu'elle se présente sous forme d'une crème pour rinçage qui contient en outre 0,1 à 5,0% en poids de tensio-actif cationique.

6. Procédé de préparation d'un sel de zinc et de pyridinethione selon la revendication 1, caractérisé en ce que l'on fait réagir de la 1-hydroxy-2-pyridinethione ou un sel hydrosoluble de celle-ci

avec un sel de zinc hydrosoluble dans un milieu tensio-actif aqueux, la température de réaction étant d'au moins 20°C.

7. Procédé selon la revendication 6, caractérisé en ce que le composé de pyridinethione est un sel de métal alcalin de la pyridinethione et en ce que la concentration de tensio-actif est le 1 à 24% en poids par rapport au total du mélange réactionnel.

8. Procédé selon la revendication 6 ou 7, caractérisé en ce que le sel de zinc est présent en quantité suffisante pour assurer un excès par rapport à la quantité stoéchiométrique.

9. Procédé selon l'une quelconque des revendications 6 à 8, caractérisé en ce que l'on fait réagir le sel de sodium de la 1-hydroxy-2-pyridinethione avec le sulfate de zinc dans un milieu tensio-actif anionique à une température comprise entre 60°C et 100°C, la concentration de tensio-actif étant de 1 à 8% en poids par rapport au total du mélange réactionnel.